# EUROPEAN PATENT SPECIFICATION

(11) **EP 0 290 179 B1**
(45) Date of publication and mention of the grant of the patent: **27.01.1993**
(21) Application number: 88303706.1
(22) Date of filing: 25.04.1988
(51) Int. Cl.: C07C 37/74, C07C 39/16

(54) **Process for preparing bisphenol A**
Verfahren zur Herstellung von Bisphenol-A
Procédé de préparation de bisphénol-A

(30) Priority: 06.05.1987 JP 108964/87
(43) Date of publication of application: 09.11.1988
(73) Proprietor: MITSUI TOATSU CHEMICALS, Inc., Chiyoda-Ku Tokyo 100 (JP)
(72) Inventor: Iimuro, Shigeru, Minami-ku Nagoya-shi Aichi-ken (JP); Kitamura, Takashi, Midori-ku Nagoya-shi Aichi-ken (JP); Morimoto, Yoshio, Tokai-shi Aichi-ken (JP)
(74) Representative: Hayward, Denis Edward Peter

(56) References cited:
- DE-A- 1 118 797
- GB-A- 974 982
- GB-A- 1 274 798
- CHEMICAL ABSTRACTS, vol. 104, no. 14, 7th April 1986, page 11, abstract no. 110347s, Columbus, Ohio, US; & NL-A-84 00 062 (MITSUI PETROCHEMICAL INDUSTRIES, LTD) 01-08-1985

## Description

The present invention relates to a process for preparing high purity 2,2-bis(4-hydroxyphenyl)propane (hereinafter referred to as bisphenol A).

Bisphenol A is used as a raw material for polycarbonate resins or epoxy resins, and colourless and high purity bisphenol A is required for polycarbonate resin in particular.

Bisphenol A is prepared from acetone and excess phenol in the presence of an acidic catalyst, in some cases by the addition of an auxiliary catalyst such as sulfur compounds. The reaction mixture contains the catalyst, unreacted acetone, unreacted phenol, water formed by the reaction and other by-products of the reaction.

The by-products are mainly composed of 2-(2-hydroxyphenyl)2-(4-hydroxyphenyl)propane and also contain Dianin's compound, trisphenol, polyphenol and undesirable coloured substances. These by-products detract from the properties of resin prepared from bisphenol A.

In a process for recovering high purity bisphenol A from the reaction mixture, the catalyst, water and a small amount of phenol are removed from the reaction mixture. The residual liquid mixture is cooled to crystallise the adduct of bisphenol A and phenol. The crystals are separated from the mother liquor containing the by-products of the reaction and then bisphenol A is recovered by removing phenol from the adduct.

As a process for removing phenol from the adduct of bisphenol A and phenol, various methods such as distillation, extraction, steam stripping etc. have been proposed.

For example, Japanese Patent Publication TOKKOSHO 52-42790 (1977) discloses a process for vaporizing the adduct at above 180°C for 0.1 - 30 minutes under reduced pressure and then obtaining bisphenol A by fractional condensation.

Japanese Patent Publication TOKKOSHO 36-23335 (1961) teaches a process for heating the adduct at 50°C or more with use of a solvent having a boiling point of at least 50°C and dissolving the phenol part alone in the solvent.

A distillation process is generally used because it is carried out with simple equipment and without other solvents or water. For example, Japanese Patent Publication TOKKOSHO 56-13700 (1981) describes a process for distilling off phenol under reduced pressure, then removing decomposable low-boiling fractions in a column and successively obtaining bisphenol A by distillation. In addition, there are disclosed processes for obtaining bisphenol A, after removing phenol under reduced pressure, by conducting the distillation in the presence of stabilisers such as aliphatic dicarboxylic acid esters [Japanese Patent Publication TOKKOSHO 45-22539 (1970)], glycols [Japanese Patent Publication TOKKOSHO 45-3925 (1970)] and propylene glycol or epoxy resin [Japanese Patent Publication TOKKOSHO 56-1297 (1981)].

In the process of distilling the adduct of bisphenol A and phenol and obtaining phenol from the top of column and bisphenol A from the bottom respectively, the adduct crystals or their resulting melt liquid are required to be heated above a specific temperature prior to being fed into the distillation column. High temperatures are required for completely eliminating phenol. For example, Japanese Patent Publication TOKKOSHO 50-12428 (1975) teaches that desirable heating temperatures are up to 205°C, e.g. the temperature at which phenol crystals are subjected to stripping.

The present inventors have found that bisphenol A solidifies in the distillation column and finally the operation becomes impossible when the temperature of the adduct crystals or their melt liquid which are fed to the distillation column is lower than a specific temperature. Therefore the inventors have extensively investigated in order to overcome the above problem. As a result, if the operating pressure is at least 50 Torr (6666 Pa), it has been found that to prevent solidification, the melt liquid of the adduct should be fed to the distillation column at not less then 120°C or the bottom temperature of the column should be maintained at round about 180°C. Under these conditions, however, substantial amounts of phenol remain so as to yield only substantially useless bisphenol A. On the other hand, when the operating pressure is less than 50 Torr (6666 Pa) solidification can be prevented by maintaining the temperature of the feed at not less than 205°C. A column having a diameter of several centimetres such as is used in the laboratory can be continuously operated by external heating, even though the feed temperature or the bottom temperature is outside of the above range. External heating of the column, however, has been found not to be practical for industrial scale equipment.

More importantly, treatment of bisphenol A at too high a temperature causes undesirable decomposition, and the undecomposed bisphenol A obtained from the bottom of the column has been found to be useless as a product whichever of the above processes is employed.

The object of this invention is therefore to provide a process for removing phenol from the adduct of bisphenol A and phenol without exposing bisphenol A to temperatures higher than needed.

As a result of the work of the present inventors, it has been found that, in the process of distilling off phenol from the top of a distillation column and recovering 2,2-bis(4-hydroxyphenyl)propane from its bottom, recycling of a part of the bottom liquid with the feed stream makes possible the efficient removal of phenol and continuous operation of the distillation column for a long period of time.

Accordingly, the process of the present invention for preparing 2,2-bis(4-hydroxyphenyl)propane comprises:
a) feeding an adduct of 2,2-bis(4-hydroxyphenyl)propane and phenol in the form of crystals, melt liquid or a mixture thereof to a distillation column; and
b) distilling the adduct and removing phenol from the top of the distillation column and a stream containing 2,2-bis(4-hydroxyphenyl)propane from the bottom of the distillation column; and is characterised by
c) recycling a portion of the stream containing 2,2-bis(4-hydroxyphenyl)propane removed from the bottom of the distillation column by combining said portion with the adduct being fed to the distillation column.

The adduct of phenol and bisphenol A which is used as the feed material may be, for example, the adduct directly obtained from the reaction product, adduct crystals prepared by concentrating the filtrate after removing the separated adduct from the reaction mixture, and furthermore crystals obtained by recrystallising a mixture of crude bisphenol A and phenol.

Such adduct can be prepared, for example, by conducting a condensation reaction of phenol and acetone in the presence of a hydrochloric acid catalyst, distilling off hydrochloric acid, water and a small amount of phenol from the reaction product and cooling the residual mass. The adduct can also be provided by directly cooling the effluent from a fixed bed reactor packed with cation exchange resin. Furthermore, as disclosed in Japanese Laid-Open Patent TOKKAISHO 51-91240 (1976), the adduct can also be crystallised by adding water to a mixture of bisphenol A and phenol prior to cooling.

According to the process of this invention, any type of distillation column such as a packed column or a plate column may be used for removing phenol. External heating is not needed so long as heat loss is avoided. When both feed components are being simultaneously fed to the column in the process of this invention, they may be mixed prior to or directly after entering the column.

The recycled liquid should preferably be heated in order to supply the heat quantity required for evaporating phenol from the adduct. The distillation column can be operated at an internal liquid temperature in the range of 160 - 200°C and under a pressure in the range of 10 - 100 Torr (1333 to 13332 Pa).

The bisphenol A obtained after removing the phenol according to this process may be used as product as it is or after further subjecting it to purification or refining in other steps.

A process according to this invention will now be described in more detail with reference to the accompanying drawings, following which some specific examples will be given.

In the drawings:-
Figure 1 is a flow diagram illustrating a process according to the invention,
Figure 2 is a flow diagram illustrating a process that is, by contrast, outside of the scope of this invention.

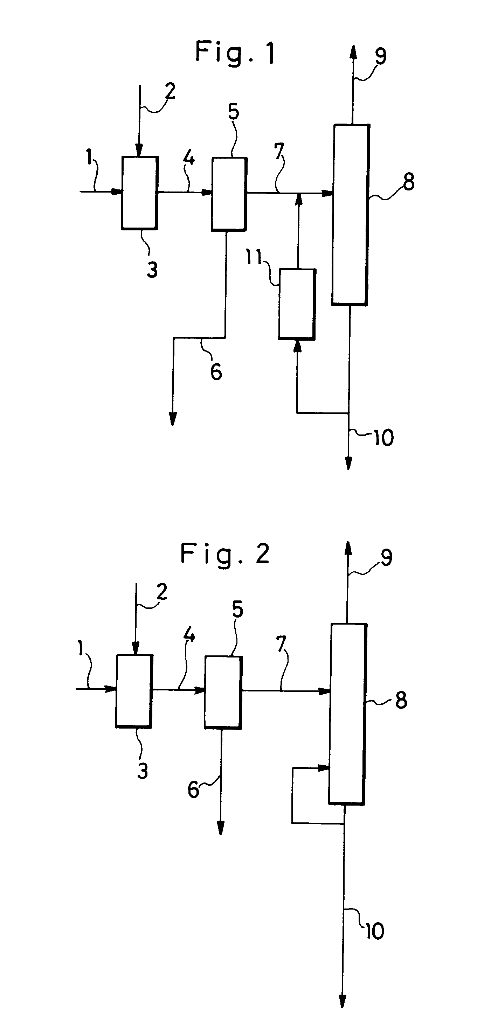

Referring to Figure 1, a mixture (1) consisting of bisphenol A, phenol and impurities is cooled in a crystallising vessel (3) with the addition of water (2) to crystallise the adduct of bisphenol A and phenol.

In the next step, the resulting slurry (4) of adduct is charged into a separator (5) to separate the adduct (7) from the mother liquid (6). The adduct (7) is then fed to a dephenolation column (8). Phenol (9) is removed from the top of the column and bisphenol A (10) containing a small amount of phenol is obtained from the bottom of the column.

A part of the bisphenol A (10) is recycled through a heater (11) to be reintroduced into the column along with the feed (7) and the remaining portion is removed as product.

In order to emphasise that, in the process of this invention, the recycled portion from the bottom of the column is reintroduced to mix with the feed (7), Figure 2 illustrates a conventional way of operating such a column with a portion of the bottom stream reintroduced into a reboil section at the bottom of the column. In Figure 2, the reference numerals 1 - 10 mean the same as in Figure 1.

By the process of this invention, phenol can readily be removed from the adduct of bisphenol A and phenol and the dephenolation column can be operated continuously for a long period of time. Good quality products can be obtained without exposing bisphenol A to unnecessarily high temperatures.

### EXAMPLES

This invention will be further illustrated by a specific example and a comparative example.

### Example

In Figure 1, a mixture (1) consisting of bisphenol A, phenol and impurities was charged into a crystallising vessel (3) at the rate of 400 kg/hour and at the same time water (2) was added at the rate of 50 kg/hour. The resulting slurry (4) of the adduct of bisphenol A and phenol was charged into a separator (5) to separate the adduct (7) from mother liquid (6). The adduct (7) was heated to 120°C, melted and fed at the rate of 160 kg/hour to a dephenolation column (8) having an internal diameter of 30cm. A part of the bottom liquid from the dephenolation column was heated to 190°C in a heater (11) and fed back to the column together with the adduct (7). The phenol (9) was mostly removed by operating at 15 Torr (2000 Pa), 170°C and the bisphenol A (10) which was taken out at the bottom of the column had a phenol content of 2% or less. No plugging of the column occurred and stable operation continued for a year.

### Comparative Example

In Figure 2, the same melt liquid of adduct (7) as in Example 1 was fed to the dephenolation column at temperatures of 150°C, 180°C and 210°C. Bisphenol A (10), however, was not recycled with the feed according to the process of this invention, but was re-circulated within the bottom reboiler section only. Crystals were observed to grow gradually from a position below the feed location and the column was plugged to stop the operation respectively after 2 hours, 5 hours and 12 hours at the three different feed temperatures that were tried.

## Claims

1. A process for preparing 2,2-bis(4-hydroxyphenyl)propane comprising:
a) feeding an adduct of 2,2-bis(4-hydroxyphenyl)propane and phenol in the form of crystals, melt liquid or a mixture thereof to a distillation column; and
b) distilling the adduct and removing phenol from the top of the distillation column and a stream containing 2,2-bis(4-hydroxyphenyl)propane from the bottom of the distillation column; characterised by
c) recycling a portion of the stream containing 2,2-bis(4-hydroxyphenyl)propane removed from the bottom of the distillation column by combining said portion with the adduct being fed to the distillation column.

2. A process according to claim 1, wherein said recycled portion is combined with the adduct prior to being fed into the distillation column.

3. A process according to claim 1, wherein said recycled portion is fed to the distillation column and combined with the incoming adduct directly thereafter.

4. A process according to any preceding claim, wherein said recycled portion is heated prior to being combined with the adduct.

5. A process according to any preceding claim, wherein the distillation column is operated at a temperature of from 160°C to 200°C.

6. A process according to any preceding claim, wherein the distillation column is operated at a pressure of from 10 to 100 Torr (1333 to 13332 Pa).

7. A process according to any preceding claim, wherein the distillation column is a packed column.

8. A process according to any preceding claim, wherein the distillation column is a plate column.

9. A process according to any preceding claim, wherein the stream containing 2,2-bis(4-hydroxyphenyl)propane removed from the bottom of the distillation column contains less than 2% by weight of phenol.

10. A process according to any preceding claim, wherein the adduct is fed to the distillation column in the form of crystals.

11. A process according to any preceding claim, wherein the adduct is fed to the distillation column in the form of melt liquid.

## Patentansprüche

1. Verfahren zur Herstellung von 2,2-bis-(4-Hydroxyphenyl)-Propan, bestehend aus den Schritten:
a) Zuführen eines Addukts von 2,2-bis-(4-Hydroxyphenyl)-Propan und Phenol in Form von Kristallen, Schmelzflüssigkeit oder einer Mischung davon zu einer Destillationssäule; und
b) Destillieren des Addukts und Entnehmen des Phenols aus der Spitze der Destillationssäule und eines Stroms mit 2,2-bis-(4-Hydroxyphenyl)-Propan aus dem Boden der Destillationssäule,
dadurch gekennzeichnet, daß
c) ein Teil des aus dem Boden der Destillationssäule entnommenen Stroms mit 2,2-bis-(4-Hydroxyphenyl)-Propan dadurch Kombinieren dieses Teils mit dem der Destillationssäule zugeführten Addukt wieder in den Kreislauf geführt wird.

2. Verfahren nach Anspruch 1, wobei besagter rückgeführter Teil vor der Zuführung zur Destillationssäule mit dem Addukt kombiniert wird.

3. Verfahren nach Anspruch 1, wobei besagter rückgeführter Teil der Destillationssäule zugeführt wird und direkt danach mit dem eintreffenden Addukt kombiniert wird.

4. Verfahren nach einem der vorhergehenden Ansprüche, wobei besagter zurückgeführter Teil vor der Kombination mit dem Addukt erwärmt wird.

5. Verfahren nach einem der vorhergehenden Ansprüche, wobei die Destillationssäule bei einer Temperatur von 160°C bis 200°C betrieben wird.

6. Verfahren nach einem der vorhergehenden Ansprüche, wobei die Destillationssäule bei einem Druck von 10 bis 100 Torr (1333 bis 13332 Pa) betrieben wird.

7. Verfahren nach einem der vorhergehenden Ansprüche, wobei die Destillationssäule eine Füllkörpersäule ist.

8. Verfahren nach einem der vorhergehenden Ansprüche, wobei die Destillationssäule eine Plattensäule ist.

9. Verfahren nach einem der vorhergehenden Ansprüche, wobei der aus dem Boden der Destillationssäule entnommene Strom mit 2,2-bis-(4-Hydroxyphenyl)-Propan weniger als 2 Gew.-% Phenol enthält.

10. Verfahren nach einem der vorhergehenden Ansprüche, wobei das Addukt der Destillationssäule in Form von Kristallen zugeführt wird.

11. Verfahren nach einem der vorhergehenden Ansprüche, wobei das Addukt der Destillationssäule in Form von Schmelzflüssigkeit zugeführt wird.

## Revendications

1. Procédé pour préparer du 2,2-bis(4-hydroxyphényl)-propane, comprenant:
a) l'amenée d'un additif à base de 2,2-bis(4-hydroxyphényl-propane et de phénol sous forme de cristaux, de liquide en fusion ou d'un mélange de ces derniers, à une colonne de distillation; et
b) la distillation de l'additif et extraction du phénol par le haut de la colonne de distillation, et d'un courant contenant du 2,2-bis(4-hydroxyphényl)-propane par le bas de la colonne de distillation; caractérisé par:
c) le recyclage d'une partie du courant contenant du 2,2-bis(4-hydroxyphényl)-propane retiré par le base de la colonne de distillation, en combinant ladite partie à l'additif amené à la colonne de distillation.

2. Procédé selon la revendication 1, dans lequel ladite partie recyclée est combinée à l'additif, avant d'être amenée dans la colonne de distillation.

3. Procédé selon la revendication 1, dans lequel ladite partie recyclée est amenée à la colonne de distillation et est combinée à l'additif introduit, directement après.

4. Procédé selon l'une quelconque des revendications précédentes, dans lequel ladite partie recyclée est chauffée avant d'être combinée à l'additif.

5. Procédé selon l'une quelconque des revendications précédentes, dans lequel la colonne de distillation est mise en fonctionnement à une température allant de 160°C à 200°C.

6. Procédé selon l'une quelconque des revendications précédentes, dans lequel la colonne de distillation est mise en fonctionnement à une température allant de 1333 à 1332 Pa (10 à 100 Torr).

7. Procédé selon l'une quelconque des revendications précédentes, dans lequel la colonne de distillation est une colonne garnie.

8. Procédé selon l'une quelconque des revendications précédentes, dans lequel la colonne de distillation est une colonne plate.

9. Procédé selon l'une quelconque des revendications précédentes, dans lequel le courant contenant du 2,2-bis(4-hydroxyphényl)-propane retiré par le haut de la colonne de distillation en contient une valeur inférieure à 2 % en poids de phénol.

10. Procédé selon l'une quelconque des revendications précédentes, dans lequel l'additif est amené à la colonne de distillation se présentant sous forme de cristaux.

11. Procédé selon l'une quelconque des revendications précédentes, dans lequel l'additif est amené à la colonne de distillation sous forme de liquide en fusion.
